# FASCICULE DE BREVET EUROPEEN

(11) **EP 1 352 643 B1**
(45) Date de publication et mention de la délivrance du brevet: **09.08.2006**
(21) Numéro de dépôt: 03290632.3
(22) Date de dépôt: 13.03.2003
(51) Int. Cl.: A61K 8/63, A61Q 19/08

(54) **Utilisation d'une sapogénine ou d'un extrait naturel en contenant, pour lisser les rides et ridules d'expression**
Verwendung von einem Sapogenin oder einem Sapogenin-enthaltend Extrakt, zur Glättung von feinen Linien und Falten
Use of a sapogenin or a sapogenin containing natural extract, to smooth out fine lines and wrinkles

(30) Priorité: 12.04.2002 FR 0204611
(43) Date de publication de la demande: 15.10.2003
(73) Titulaire: L'ORÉAL, 75008 Paris (FR)
(72) Inventeur: Besne, Isabelle, 92800 Puteaux (FR)
(74) Mandataire: Renard, Emmanuelle

(56) Documents cités:
- FR-A- 2 802 412
- FR-A- 2 803 513
- FR-A- 2 811 561
- FR-A- 2 811 568
- FR-A- 2 811 569
- US-A- 5 723 149
- US-B1- 6 331 535

## Description

La présente invention concerne un procédé cosmétique pour lisser les rides et ridules d'expression, comprenant l'application topique sur les zones du visage ou du front marquées par des rides et ridules d'expression et/ou sur les personnes présentant des rides et ridules d'expression d'une composition comprenant au moins une sapogénine, ou un extrait végétal en contenant, ou un ester de sapogénine dans un milieu physiologiquement acceptable. Elle concerne également l'utilisation d'une sapogénine, d'un extrait végétal en contenant, ou d'un ester de sapogénine, dans une composition adaptée à une application topique sur la peau, comme agent destiné à lisser les rides et ridules d'expression.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané.

Bien que ces traitements permettent d'agir sur les rides et ridules dues au vieillissement chronologique ou intrinsèque, ainsi que de celles dues au photo-vieillissement, ils n'ont pas d'effet sur les rides et ridules d'expression, lesquelles nécessitent une intervention sur la composante contractile musculaire des rides présente dans la peau.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'Iris pallida (FR-2 746 641).

Il reste toutefois le besoin de disposer de composés d'origine naturelle efficaces pour lisser ou estomper les rides et ridules d'expression.

Or, la Demanderesse a découvert avec étonnement que les sapogénines permettaient de satisfaire ce besoin.

Les sapogénines sont des composés résultant de l'hydrolyse acide (généralement en présence d'acide chlorhydrique ou sulfurique en milieu aqueux bouillant) ou enzymatique (généralement pendant environ 5 jours à environ 37°C), habituellement suivie d'une extraction par un solvant organique apolaire, des saponosides qui sont des hétérosides fonctionnalisés, de poids moléculaire très élevé, présents dans les tubercules de certaines plantes. Comme sources de saponosides, on pourra citer : les plantes de la famille des dioscoréacées comme *Dioscorea composita, Dioscorea deltoides, Dioscorea floribunda, Dioscorea sylvatica, Dioscorea spiculiflora et Dioscorea villosa*, qui sont riches en dioscine ; les plantes de la famille des Agaves et les plantes de la famille des Liliacées, dont le yucca.

Parmi les sapogénines que l'on peut obtenir à partir des plantes précitées, la diosgénine a déjà été décrite comme anti-inflammatoire (Yamada et al., *Am. J. Physiol*., 273:G355-G364, 1997), comme actif amincissant par son action sur les adipocytes (WO 00/30603), et comme agent anti-microbien utilisable dans le traitement de diverses pathologies à composante infectieuse dont l'acné et la dermatite séborrhéique (DE-198 41 795).

En outre, des extraits de *Dioscorea tokoro*, qui renferment de la dioscine, ont par ailleurs été décrits comme efficaces pour hydrater la peau et ainsi l'assouplir. Ainsi, le document JP-10 194 947 divulgue un extrait de *Dioscorea tokoro* préparé par extraction à l'aide d'eau, d'alcool ou d'acétone à une température comprise entre 0 et 50°C, de préférence de 0°C. Cet extrait est décrit comme utile pour améliorer la souplesse et l'hydratation de la peau en raison des glycoprotéines qu'il contient, notamment en vue de prévenir la formation de rides. Une diminution de la profondeur des rides de la patte d'oie est en outre observée.

De son côté, le document JP-2000 143 488 divulgue une composition pour prévenir et améliorer le dessèchement cutané, comprenant un extrait de plante choisi parmi une longue liste, dont *Dioscorea tokoro maquino* et *Dioscorea gracillima miq*., qui sont des sources de saponosides. Là encore, le procédé d'extraction comprend simplement l'immersion de la plante dans un solvant à température ambiante, suivie d'étapes de filtration et de séchage.

Ainsi, les procédés d'extraction décrits dans ces documents ne permettent pas d'obtenir des compositions contenant des sapogénines (dont la diosgénine), dans la mesure où ils ne comprennent pas d'étape d'hydrolyse acide ou enzymatique. Les compositions décrites ne contiennent donc que des saponosides (dont la dioscine).

Par ailleurs, la demande FR-00/16074 (non publiée) divulgue l'utilisation des sapogénines comme agents anti-collagénase, dans des compositions cosmétiques destinées notamment à prévenir la formation des rides.

Il n'est pas suggéré que ces compositions puissent avoir un effet de lissage des rides et ridules existantes, a fortiori des rides et ridules d'expression.

Le document **FR-2 811 569** divulgue une composition cosmétique renfermant la DHEA ou un précurseur qui peut être une sapogénine, en combinaison avec un caroténoïde. La composition est notamment destinée à traiter les signes du vieillissement cutané, en particulier du vieillissement actinique (dû aux UV), dont les rides et ridules.

Le document **FR-2 803 513** divulgue l'utilisation de la DHEA ou de ses précurseurs tels que les sapogénines, pour améliorer l'aspect papyracé de la peau. Cet aspect de la peau se caractérise par un ensemble de plis fins et aigus semblables à un papyrus ou à une feuille de papier à cigarette, qui recouvre en particulier les joues. Les rides d'expression que la présente invention vise à combattre sont au contraire nettement caractérisées par des sillons marqués disposés horizontalement sur le front et verticalement dans l'espace inter-sourcillier.

Le document **FR-2 802 412** divulgue une crème anti-rides qui agit en stimulant la micro-circulation et comprend notamment (parmi bien d'autres constituants) des sapogénines en tant qu'agent anti-inflammatoire et vaso-constricteur.

Le document **US-5,723,149** divulgue une composition contenant des sapogénines de Medicago (luzerne), en particulier l'hédéragénine, et son utilisation dans le traitement des signes du vieillissement cutané. L'effet anti-âge est obtenu par une stimulation du renouvellement épidermique via une prolifération des kératinocytes et par l'augmentation de la synthèse de collagène.

La présente invention a donc pour objet un procédé cosmétique pour lisser les rides et ridules d'expression, comprenant l'application topique sur les zones du visage ou du front marquées par des rides et ridules d'expression et/ou sur les personnes présentant des rides et ridules d'expression d'une composition comprenant au moins une sapogénine, ou un extrait végétal en contenant, ou un ester de sapogénine dans un milieu physiologiquement acceptable.
Elle a aussi pour objet l'utilisation d'une sapogénine, d'un extrait végétal en contenant, ou d'un ester de sapogénine, dans une composition adaptée à une application topique sur la peau, comme agent destiné à lisser les rides et ridules d'expression.

Les rides et ridules concernées sont celles situées au niveau du front, en particulier la ride dite du lion, située au niveau de la glabelle, dans l'espace inter-sourcillier, et/ou disposées horizontalement sur le front.

La sapogénine mise en oeuvre selon l'invention peut être choisie parmi : la diosgénine, l'hécogénine, la smilagénine, la sarsapogénine, la tigogénine, la yamogénine et la yuccagénine.

La présente invention concerne toutefois plus particulièrement la diosgénine. La diosgénine est notamment disponible auprès de la société SABINSA sous la dénomination commerciale Diosgenin® .

Par "extraits végétaux", on entend tout extrait végétal renfermant une ou plusieurs sapogénines, après traitement destiné à hydrolyser les saponosides, tel qu'un extrait de Dioscoréacée, qui contient de la diosgénine, ou un extrait de feuille d'agave contenant de l'hécogénine et de la tigogénine, ou encore un extrait de Liliacée, en particulier du genre Smilax ou Yucca, contenant de la smilagénine et de la sarsapogénine, tel qu'un extrait de racine de salsepareille.

Ainsi, la diosgénine peut être extraite des tubercules de certaines Dioscoréacées, comme indiqué précédemment, par un procédé comprenant successivement : l'hydrolyse à chaud des hétérosides en milieu acide minéral (éventuellement après fermentation et séchage des tubercules) ; et la filtration de la fraction insoluble, qui est ensuite neutralisée, lavée et traitée par un solvant apolaire. D'autres procédés d'extraction sont cependant utilisables.

Les variétés de Dioscoréacées renfermant de la dioscine, précurseur de diosgénine, sont listées dans la publication de Franklin W. MARTIN, The Species of Dioscorea Containing Sapogenin, Econ. Bot., Vol. 23, 373-384 (1969) à laquelle on pourra se reporter.

Parmi celles-ci, on peut citer les espèces *Dioscorea composita*, *Dioscorea deltoides, Dioscorea floribunda, Dioscorea sylvatica, Dioscorea spiculiflora, Dioscorea opposita, Dioscorea mexicana et Dioscorea villosa*. L'extrait utilisé selon l'invention est préparé préférentiellement à partir de matériel végétal provenant des espèces *Dioscorea villosa* et *Dioscorea opposita*. Un tel extrait est notamment disponible auprès de la société ACTIVE ORGANICS sous les dénominations commerciales Actigen Y® et Actiphyte Mexican Wild Yam® . Il peut en variante être obtenu auprès de la société OSST sous la dénomination commerciale Wild Yam P.E. ® ou auprès de la société ALBAN MÜLLER sous la dénomination commerciale Extrait sec igname sauvage® .

Comme esters de sapogénines, on citera en particulier l'acétate d'hécogénine.

La quantité de sapogénine ou ester utilisable selon l'invention est bien entendu fonction de l'effet recherché et peut donc varier dans une large mesure.

Pour donner un ordre de grandeur, on peut utiliser la sapogénine, ou son ester, en une quantité représentant de 0,001% à 5% du poids total de la composition, préférentiellement en une quantité représentant de 0,01% à 1,5% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses.

Cette composition peut se présenter sous toutes les formes galéniques normalement utilisées dans le domaine cosmétique , et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des sapogénines ou des extraits végétaux selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants ; les agents tenseurs ; les agents antipollution et/ou anti-radicalaire ; les agents agissant sur la microcirculation ; les agents agissant sur le métabolisme énergétique des cellules ; et leurs mélanges.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.); les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les filtres inorganiques sont de préférence constitués d'oxyde de zinc et/ou de dioxyde de titane, de préférence de taille nanométrique, éventuellement enrobés d'alumine et/ou d'acide stéarique.

L'invention sera maintenant illustrée par les exemples non limitatifs suivants. Dans ces exemples, les quantités sont indiquées en pourcentage pondéral.

### EXEMPLES

### Exemple 1 : Mise en évidence de l'effet myorelaxant des sapogénines

L'acétate d'hécogénine et la diosgénine, fournis par SIGMA, ont été testées sur un modèle de jonction nerf / muscle (plaque motrice) obtenu dans une préparation nerf phrénique / diaphragme isolé de rat (muscle strié) (Pollard B.J. et al, Br.J. Anaesth., 1988, 61, 419-424).

Le nerf phrénique et le diaphragme sont soigneusement isolés et placés dans une cuve de 50 ml remplie de liquide de survie (liquide de Krebs Henseleit) maintenu à une température de 37°C et oxygéné à l'aide d'un mélange oxygène/CO₂ (95/5).

Les variations de tension du diaphragme sont enregistrées avec une pré charge initiale de plusieurs grammes.

Après une période de relaxation de 30 mn, le diaphragme est stimulé indirectement par l'intermédiaire du nerf phrénique.

Sur chaque préparation, l'effet des produits à tester a été évalué sur les contractions induites par stimulation indirecte via stimulation sur le nerf phrénique (0.1 à 0.5 volts, 0.3 ms, 0.1 Hz) aux concentrations croissantes et cumulées de 10⁻¹⁰ M à 10⁻⁵ M.

Les résultats obtenus dans le modèle de plaque motrice avec les sapogénines sont présentés dans le tableau ci-dessous :

| PRODUIT | CONCENTRATION | % INHIBITION DES CONTRACTIONS | IC50* |
|---|---|---|---|
| Diosgénine | 10⁻⁵ M | 100% | 10⁻⁶ M |
| Acétate d'hécogénine | 10⁻⁵ M | 100% | 2,5 x 10⁻⁶ M |

| | | | |
|---|---|---|---|
| *Concentration pour obtenir 50% de l'effet inhibiteur | | | |

A titre de comparaison, à la concentration de 10⁻⁴M, l'Alvérine inhibe complètement les contractions induites par les stimulations indirectes. En revanche, elle n'a pas d'effet sur ces contractions à la concentration de 10⁻⁵ M.

Ainsi, la diosgénine et l'acétate d'hécogénine sont des agents myorelaxants plus efficaces que l'alvérine et utiles à cet effet dans le lissage des rides et ridules d'expression.

### Exemple 2 : Compositions cosmétiques

Ces compositions sont préparées de manière classique pour l'homme du métier. Les quantités données dans ces exemples sont indiquées en pourcentages pondéraux.

### A. Crème

| | | |
|---|---|---|
| Mélange d'alcool cétéarylique et de glucoside cétéarylique | | 4 % |
| Mélange de stéarate de glycéryle et stéarate de PEG-100 | | 1 % |
| Alcool cétylique | | 0,5 % |
| Benzoate d'alkyles en C₁₂₋₁₅ | | 2 % |
| Polyisobutène hydrogéné | | 5 % |
| Vaseline | | 2 % |
| Huiles végétales | | 3,5 % |
| Cire de silicone | | 2 % |
| Silicone volatile | | 5 % |
| Extrait de *Padina pavonica* | | 1 % |
| Extrait d'igname sauvage | | 0,7 % |
| Extrait de soja riche en isoflavones | | 1 % |
| PEG-20 | | 1 % |
| Alcool | | 3 % |
| Glycérol | | 7 % |
| Gélifiants | | 1,5 % |
| Filtres UVA et UVB | | 12,5 % |
| Neutralisant | | 1,5 % |
| Conservateurs | | QS |
| Eau | QSP | 100 % |

### B. Crème pour peaux sèches

| | |
|---|---|
| Octyl dodécanol | 0,5 % |
| Acide stéarique | 3,0 % |
| Mélange de mono-stéarate de glycéryle et de stéarate de polyéthylène glycol (100 OE) | 2,0 % |
| Stéarate de polyéthylène glycol (20 OE) | 1,0 % |
| Conservateurs | QS |
| Polyisobutène hydrogéné | 6,0 % |
| Néopentanoate d'isostéaryle | 3,5 % |
| Gluconate de manganèse | 0,05 % |
| Glycérine | 3,0 % |
| Huiles végétales | 7,0 % |
| Charges | 6,0 % |
| Alcools gras | 1,0 % |
| Corps gras siliconés | 13,0 % |
| Extrait peptidique de graines de pois | 2,0 % |
| Céramides | 0,1 % |
| Acide poly(acrylamidométhyl propane sulfonique) | 1,0 % |
| Extrait de rhizome d'igname sauvage (à 3% de diosgénine) | 2,0 % |

Ces crèmes sont destinées à être appliquées sur le visage et le front pour atténuer les rides et ridules d'expression.

### C. Sérum

| | | |
|---|---|---|
| Octyldodécanol | | 0,4 % |
| Acétate d'hécogénine | | 0,1 % |
| Conservateurs | | QS |
| Butylène glycol | | 1 % |
| Hydroxyde de sodium | | 0,7 % |
| Glyceryl stearate et PEG-100 stearate | | 0,3 % |
| PEG-60 hydrogenated castor oil | | 1 % |
| Ethanol | | 5 % |
| EDTA disodique | | 0,05 % |
| Gélifiants | | 2,5 % |
| Polysilicone-8 | | 1 % |
| Hyaluronate de sodium | | 0,1 % |
| Glycérine | | 4 % |
| Dimethicone et dimethiconol | | 3 % |
| Acide n-octanoyl-5-salicylique | | 0,2 % |
| Protéine de soja | | 1 % |
| Extrait d'algue brune *Padina pavonica* | | 1 % |
| Acide citrique | | 1,2 % |
| Eau | qsp | 100 % |

Ce sérum est destiné à être appliqué autour de la bouche pour atténuer les rides et ridules verticales.

### D. Crème contour des yeux

| | | |
|---|---|---|
| Glycérine | | 12 % |
| Extrait de racine de *Dioscorea villosa* | | 0,35 % |
| Extrait de racine de *Ruscus aculeatus* | | 0,1 % |
| Cyclohexasiloxane | | 4 % |
| Diméthicone et dimethiconol | | 0,5 % |
| Huiles végétales | | 9 % |
| Alcools gras | | 1,7 % |
| Myristyl myristate | | 1 % |
| PEG-40 stearate et glyceryl stearate | | 0,9 % |
| Sorbitan tristearate | | 0,2 % |
| Potassium cetyl phosphate | | 0,7 % |
| Carbomer | | 0,5 % |
| Silice | | 2 % |
| Escine | | 0,1 % |
| Conservateurs | | QS |
| Triéthanolamine | | 0,5 % |
| EDTA disodique | | 0,2 % |
| Eau | qsp | 100 % |

Cette crème, utilisée quotidiennement sur le contour des yeux, permet d'estomper les rides et ridules de la patte d'oie.

## Revendications

1. Procédé cosmétique pour lisser les rides et ridules d'expression situées au niveau du front et/ou dans l'espace inter-sourcillier, comprenant l'application topique sur les zones du visage ou du front marquées par lesdites rides et ridules d'expression d'une composition comprenant au moins une sapogénine, ou un extrait végétal en contenant, ou un ester de sapogénine, dans un milieu physiologiquement acceptable.

2. Utilisation d'une sapogénine, d'un extrait végétal en contenant, ou d'un ester de sapogénine, dans une composition adaptée à une application topique sur la peau, comme agent destiné à lisser les rides et ridules d'expressionsituées au niveau du front et/ou dans l'espace inter-sourcillier.

3. Procédé selon la revendication 1 ou utilisation selon la revendication2, **caractérisé en ce que** ladite sapogénine est choisie parmi : la diosgénine, l'hécogénine, la smilagénine, la sarsapogénine, la tigogénine, la yamogénine et la yuccagénine.

4. Procédé ou utilisation selon la revendication 3, **caractérisé en ce que** la sapogénine est la diosgénine.

5. Procédé selon la revendication 1 ou utilisation selon la revendication 2, **caractérisé en ce que** ledit extrait végétal est choisi parmi les extraits de rhizomes de *Dioscorea composita, Dioscorea deltoides, Dioscores floribunda, Dioscorea sylvatica, Dioscorea spiculiflora, Dioscorea opposita, Dioscorea mexicana* et *Dioscorsa villosa.*

6. Procédé selon la revendication 1 ou utilisation selon la revendication 2, **caractérisé en ce que** ledit ester de sapogénine est l'acétate d'hécogénine.

7. Procédé selon la revendication 1 ou utilisation selon l'une quelconque des revendications 2 à 6, **caractérisé en ce que** ladite sapogénine ou son ester représente de 0,1 à 1,5% du poids total de la composition.

8. Procédé selon la revendication 1 ou utilisation selon l'une quelconque des revendications 2 à 7, **caractérisé en ce que** ladite composition renferme en outre au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants ; les agents tenseurs ; les agents antipollution et/ou anti-radicalaire ; les agents agissant sur la microcirculation ; les agents agissant sur le métabolisme énergétique des cellules ; et leurs mélanges.

## Claims

1. Cosmetic method of smoothing out expression wrinkles and fine lines located on the forehead and/or in the space between the eyebrows, comprising topical application, to the areas of the face or of the forehead that are marked by the said expression wrinkles and fine lines, of a composition comprising at least one sapogenin, or a plant extract containing it, or a sapogenin ester, in a physiologically acceptable medium.

2. Use of a sapogenin, of a plant extract containing it or of a sapogenin ester, in a composition that is suitable for topical application to the skin, as an agent for smoothing out expression wrinkles and fine lines located on the forehead and/or in the space between the eyebrows.

3. Process according to Claim 1 or use according to Claim 2, **characterized in that** the said sapogenin is chosen from: diosgenin, hecogenin, smilagenin, sarsapogenin, tigogenin, yamogenin and yuccagenin.

4. Process or use according to Claim 3, **characterized in that** the sapogenin is diosgenin.

5. Process according to Claim 1 or use according to Claim 2, **characterized in that** the said plant extract is chosen from extracts of rhizomes of *Dioscorea composita, Dioscorea deltoides, Dioscorea floribunda, Dioscorea sylvatica, Dioscorea spiculiflora, Dioscorea opposita, Dioscorea mexicana* and *Dioscorea villosa.*

6. Process according to Claim 1 or use according to Claim 2, **characterized in that** the said sapogenin ester is hecogenin acetate.

7. Process according to Claim 1 or use according to any one of Claims 2 to 6, **characterized in that** the said sapogenin or ester thereof represents from 0.1% to 1.5% of the total weight of the composition.

8. Process according to Claim 1 or use according to any one of Claims 2 to 7, **characterized in that** the said composition also contains at least one compound chosen from: desquamating agents; moisturizers; depigmenting or propigmenting agents; antiglycation agents; NO-synthase inhibitors; agents for stimulating the synthesis of dermal or epidermal macromolecules and/or for preventing their degradation; agents for stimulating the proliferation of fibroblasts and/or keratinocytes or for stimulating the differentiation of keratinocytes; other muscle relaxants; tensioning agents; antipollution agents and/or free-radical scavengers; agents acting on the capillary circulation; agents acting on the energy metabolism of cells; and mixtures thereof.

## Patentansprüche

1. Kosmetisches Verfahren zur Glättung von Mimikfalten und -fältchen, die sich an der Stirn und/oder im Bereich zwischen den Augenbrauen befinden, das das topische Aufbringen einer Zusammensetzung, die mindestens ein Sapogenin, einen Pflanzenextrakt, der ein Sapogenin, enthält, oder einen Sapogeninester in einem physiologisch akzeptablen Medium enthalt, auf die Bereiche des Gesichts oder der Stirn umfasst, die solche Mimikfalten und -fältchen aufweisen.

2. Verwendung eines Sapogenins, eines Sapogenin-haltigen Pflanzenextrakts oder eines Sapogeninestexs in einer Zusanunensetzung, die für eine topische Anwendung auf die Haut geeignet ist, als Wirkstoff, der dazu dient, Mimikfalten und -fältchen zu glätten, die sich auf der Stirn und/ oder im Bereich zwischen den Augenbrauen befinden.

3. Verfahren nach Anspruch 1 oder Verwendung nach Anspruch 2, **dadurch gekennzeichnet, dass** das Sapogenin ausgewählt ist unter: Diosgenin, Hecogenin, Smilagenin, Sarsapogenin, Tigogenin, Yamogenin und Yuccagenin.

4. Verfahren oder Verwendung nach Anspruch 3, **dadurch gekennzeichnet, dass** das Sapogenin das Diosgenin ist.

5. Verfahren nach Anspruch 1 oder Verwendung nach Anspruch 2, **dadurch gekennzeichnet, dass** der Pflanzenextrakt unter den Extrakten aus den Rhizomen von *Dioscorea composita, Dioscorea deltoides, Dioscorea floribunda, Dioscorea syluatica, Dioscorea spiculiflora, Dioscorea opposita, Dioscorea* mexicana und *Dioscorea villosa* ausgewählt ist.

6. Verfahren nach Anspruch 1 oder Verwendung nach Anspruch 2, **dadurch gekennzeichnet, dass** der Sapogeninester das Hecogeninacetat ist.

7. Verfahren nach Anspruch 1 oder Verwendung nach einem der Ansprüche 2 bis 6, **dadurch gekennzeichnet, dass** das Sapogenin oder der Sapogeninester in einer Menge von 0,1 bis 1,5 % des Gesamtgewichts der Zusammensetzung enthalten ist.

8. Verfahren nach Anspruch 1 oder Verwendung nach einem der Ansprüche 2 bis 7, **dadurch gekennzeichnet, dass** die Zusammensetzung ferner mindestens eine Verbindung enthält, die unter den folgenden Verbindungen ausgewählt ist; abschuppenden Wirkstoffen, Hydratisierungsmitteln; depigmentierenden Wirkstoffen oder pigmentierungsfördernden Wirkstoffen; Anti-Glykations-Wirkstoffen; NO-Synthase-Inhibitoren; Wirkstoffen, die die Synthese von Makromolekülen der Dermis oder Epidermis fördern und/oder ihre Zersetzung verhindern; Wirkstoffen, die die Proliferation von Fibroblasten und/oder Keratinocyten stimulieren oder die Differenzierung von Keratinocyten stimulieren; Myorelaxantien; straffenden Wirkstoffen; Anti-Pollutions-Wirkstoffen und/oder Radikalfängern für freie Radikale; Wirkstoffen, die die Mikrozirkulation beeinflussen; Wirkstoffen, die den Energieumsatz der Zellen beeinflussen; und deren Gemischen.
